Europäisches Patentamt

(19) European Patent Office

Office européen des brevets

(11) Publication number: **0 044 300**
**B1**

# EUROPEAN PATENT SPECIFICATION

(45) Date of publication of patent specification: **14.03.84**

(51) Int. Cl.³: **A 61 F 1/03, A 61 F 1/00**

(21) Application number: **80901726.2**

(22) Date of filing: **25.09.80**

(86) International application number:
**PCT/GB80/00148**

(87) International publication number:
**WO 81/00808 02.04.81 Gazette 81/8**

(54) ENDOPROSTHETIC BONE JOINT DEVICES.

(30) Priority: **25.09.79 GB 7933184**

(43) Date of publication of application:
**27.01.82 Bulletin 82/4**

(45) Publication of the grant of the patent:
**14.03.84 Bulletin 84/11**

(84) Designated Contracting States:
**CH DE FR LI**

(56) References cited:
**FR - A - 2 105 998**
**FR - A - 2 360 295**
**FR - A - 2 413 078**
**FR - A - 2 427 315**
**GB - A - 2 003 845**
**US - A - 3 877 080**

(73) Proprietor: **NATIONAL RESEARCH DEVELOPMENT CORPORATION**
**101 Newington Causeway**
**London SE1 6BU (GB)**

(72) Inventor: **Knight, Martin Tonbridge Norton**
**168 Richmond Road**
**London E8 3HN (GB)**

(74) Representative: **Parker, Geoffrey**
**Patent Department National Research Development Corporation 101 Newington Causeway**
**London SE1 6BU (GB)**

Courier Press, Leamington Spa, England.

## Endoprosthetic bone joint devices

Endoprosthetic bone joint devices have been the subject of considerable development during the past few decades and are now widely employed in routine clinical practice. The development in question has given rise to improvements in many respects, among which important ones are the biocompatability of the materials used in the devices, the frictional and other mechanical properties of such materials whereby the articulatory and wear characteristics of the devices have been enhanced, and the techniques for securing the devices to bones. However, considerable as the improvements have been, there is room for further improvement in most respects when viewed against the overall performance of natural healthy joints.

The object of the present invention is to afford some further improvement, which improvement centres on the promotion of anastomosis, that is to say the growth of living tissue, into a component of an endoprosthetic bone joint device. Anastomosis can be achieved by the use of porous material in the component, as discussed in FR—A— 2 413 078 and FR—A—2 105 995, but yet further improvement is possible.

According to the present invention there is provided an endoprosthetic bone joint device comprising a component of which one part is adapted for securement with bone, of which another part is adapted for mutual articulatory engagement with another integer of the joint, and which is substantially wholly porous, characterised in that the porosity in said one part is greater than that in said other part respectively to promote the formation of osseous and fibrocartilogenic material by way of anastomosis.

Preferably the desired variation in porosity involved differences in pore size in a diametrical sense, with the pores being interlinked throughout the component rather than being of a closed cell form.

The intended benefit of the presently proposed component is that tissue in-growth occurs in a fibrous form followed by ossification within the larger pores of the one part to provide secure anchorage of the component, while the tissue in the other part, and particularly at the outer surface, assumes a fibrocartilagenic form to provide enhanced articulatory properties. The anastomosis at the outer surface can be additionally promoted by the provision of grooving therein.

Suitable ranges of pore size for the one and other parts of the component are respectively 10—250 $\mu$m and from substantially zero, or 5 $\mu$m say, to 150$\mu$m, with the smaller regions of such ranges being preferred. The reason for this last preference is evident from the fact that the character of the component will progressively improve during a period of time following surgical implantation, but the component requires certain initial properties which, generally speaking, will be more satisfactorily achieved with higher density for the component. For the same reasons, the component may well be adapted to afford securement of a mechanical nature to provide some measure of stability during the anastomotic period after implantation.

A particularly important aspect of the present invention is that of the materials to be used for its implementation because the materials clearly must satisfy various requirements such as biocompatibility, strength, etc., as well as be capable of formation with the desired varying porosity. In practice it is preferred that these materials be of so-called carbon-carbon or carbon-fibre-reinforced carbon (CFRC) form. Satisfaction by this preference of some of the materials requirements is immediately evident because carbon is of proven biocompatability in a variety of forms and is of proven high strength in fibre form composites. Other benefits of the use of CFRC are less immediately evident.

The structure of bone in the region of the joints and elsewhere is not random or arbitrary but has a patterned form best designed to suit the functions of the bone. More specifically bone has a trabucellular structure which is generally orientated in the region of the joints to best sustain the loads transmitted therethrough. CFRC is produced, in very general terms, by impregnating carbon fibre material with a resin or some other binder and then pyrolising the resultant composite or by pyrolytic deposition on carbon fibre material of carbon from hydrocarbon gases. In either event, it will be appreciated that the fibrous reinforcement can be structured in a similar manner to bone to deploy the strength of the material in a correspondingly optimum manner. Moreover, such a structure can and indeed normally will, provide a complementary pore pattern of a similar nature whereby the anastomosis provides a structure which further enhances the simulation of bone.

A further benefit of CFRC is that it is normally porous and its porosity can be controlled to a satisfactory extent for the present purpose. This control involves a suitable choice of initial fibrous material in respct of fibre size, form of weave or other collective fabrication, etc., and choice of in-fill procedure. In this last respect, production of a CFRC component according to the invention suitably involves both impregnation and deposition as mentioned above. For example, the eventual component may involve production in separate parts with respectively different porosity attained by differing extents of isothermal carbon vapour deposition, which parts are then bonded with resin thereafter pyrolised, or a single component matrix of carbon fibre material and binder can be pyrolised and then subjected to thermal-gradient carbon vapour deposition.

The principal features of the invention having been described above, it is useful to illustrate the invention further, by way of example, with reference to the accompanying drawings which schematically illustrate an endoprosthetic hip joint device comprising two components according to the invention, and in which:—

Figures 1 and 2 are respective side and plan views of the acetabular component of the device; and

Figures 3 and 4 are respective corresponding views of the humeral component of the device.

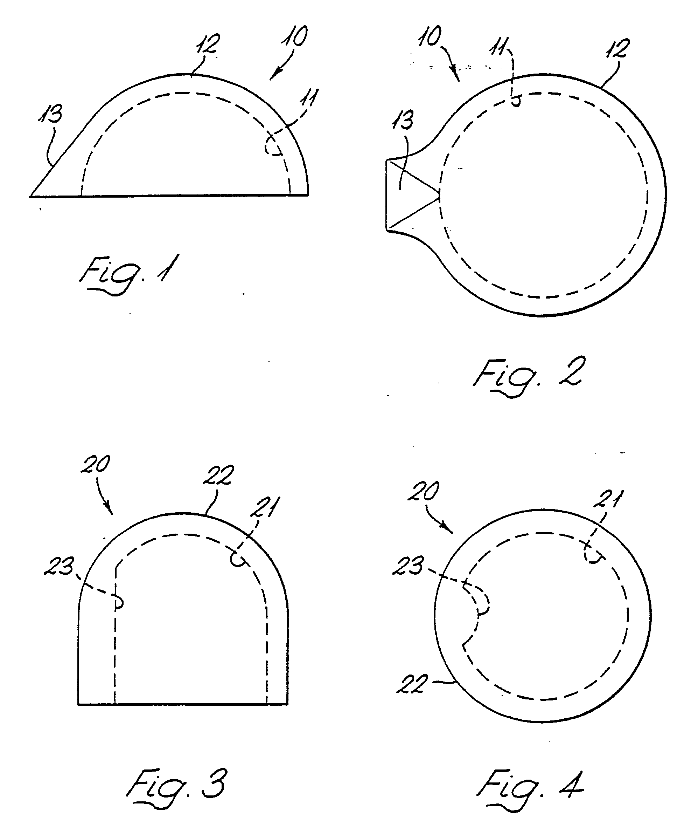

The acetabular component of Figures 1 and 2 comprises a substantially hemispherical cup body 10 with inner surface 11 and outer surface 12, the body having a key 13 formed by a projection of its outer surface. More particularly the key projection is longest at the rim of the cup where it extends radially outwardly parallel to the rim in a generally rectangular shape, as seen in Figure 2, the remainder of the key being convergently tapered partway towards the cup apex in tangential manner as seen in Figure 1. The cup is formed from CFRC as described above with greater porosity at the surface 12 than 11 where it is least. In use the cup is located in the acetabulum, with the key in the acetabular notch, a suitable seating having been prepared for the component by reaming or other preparation whereby the cup seats in a close fit with the key acting against rotation of the cup relative to the bone.

The femoral component of Figures 3 and 4 comprises a cap body 20 of an overall shape formed by a hemispherical portion leading smoothly at its rim into a right circular cylindrical projection. The inner and outer surfaces are denoted at 21 and 22, the former extending inwardly along an axially directed strip portion thereof to form a key 23 of circular arcuate transverse cross-sectional profile, and the latter being complementary in its hemispherical part with the cup 10 for mutual articulatory engagement therein. The cap is formed from CFRC as described above with greater porosity at the surface 21 than 22 where it is least. In use the cap is located over the head of the femur after suitable surgical shaping of the latter to receive the cap in a close fit with the key acting against rotation relative to the bone.

The device formed by these components is similar in general geometry and intended application to others which are already available, but these others differ in the use of metal/plastics materials combinations which do not allow anastomosis. The present invention is in fact more generally applicable in other known configurations for various joints and is in no way intended to be limited by the illustrated example.

## Claims

1. An endoprosthetic bone joint device comprising a component of which one part (12, 21) is adapted for securement with bone, of which another part (11, 22) is adapted for mutual articulatory engagement with another integer of the joint, and which is substantially wholly porous, characterised in that the porosity in said one part (12, 21) is greater than in that in said other part (11, 22) respectively to promote the formation of osseous and fibrocatilogenic material by way of anastomosis.

2. A device according to claim 1 characterised in that said porosity involves variation in pore diameter in said one and other parts within respective ranges from 10 to 250 $\mu$m and from substantially zero to 150 $\mu$m.

3. A device according to claim 1, or 2 characterised in that said component is made of a fibre reinforced material with such reinforcement being arranged to conform to a predetermined pattern therewithin which pattern simulates that of the trabecullular structure in the bone replaced by said component.

4. A device according to claim 1, 2 or 3 characterised in that said component is made of carbon-fibre-reinforced carbon.

5. A device according to claim 4 wherein said component is made from initially separate portions respectively to form said one and other parts, with said portions being subjected to respectively different extents of isothermal carbon vapour deposition to afford corresondingly different porosity, and said portions thereafter being bonded together.

6. A device according to claim 4 characterised in that said component is formed to have said varying porosity by thermal-gradient carbon vapour deposition.

7. A device according to any preceding claim characterised by a further component as aforesaid to act as said other integer.

## Patentansprüche

1. Endoprothetische Vorrichtung zur Verbindung von Knochen, enthaltend eine Komponente von der ein Teil (12, 21) dazu geeignet ist, mit Knochen fest verbunden zu werden und von der ein anderer Teil (11, 22) zur gegenseitigen, gelenkigen Verbindung mit einem anderen Teil des Gelenks geeignet und praktisch völlig porös ist, dadurch gekennzeichnet, daß die Porosität in diesem einen Teil (12, 22) größer ist als in dem anderen Teil (11, 22) und so die Bildung von Knochen- und Faserknorpelmaterial durch Anastomose zu begünstigen.

2. Vorrichtung nach Anspruch 1, dadurch gekennzeichnet, daß diese Porosität eine Variation im Porendurchmesser in dem einen und anderen Teil in Bereichen von 10 bis 250 $\mu$m bzw.

von praktisch 0 bis 150 μm umfaßt.

3. Vorrichtung nach Anspruch 1 oder 2, dadurch gekennzeichnet, daß diese Komponente aus einem faserverstärkten Material hergestellt ist, wobei diese Verstärkung so angeordnet ist, daß sie mit einem vorbestimmten Muster darin übereinstimmt und wobei dieses Muster das der trabekulären Struktur im Knochen, der durch diese Komponente ersetzt wird, nachahmt.

4. Vorrichtung nach Anspruch 1, 2 oder 3, dadurch gekennzeichnet, daß diese Komponente aus kohlenstoffaserverstärkter Kohle (CFRC) hergestellt ist.

5. Vorrichtung nach Anspruch 4, dadurch gekennzeichnet, daß diese Komponente aus anfänglich getrennten Teilen gemacht ist, die jeweils den einen und den anderen Teil bilden, wobei diese Teile jeweils unterschiedlichen Ausmaßen von isothermischer Kohledampfabscheidung unterworfen werden, um entsprechend unterschiedliche Porosität zu erzeilen und daß diese Teile danach miteinander verbunden werden.

6. Vorrichtung nach Anspruch 4, dadurch gekennzeichnet, daß diese Komponente so ausgebildet ist, daß sie die wechselnde Porosität durch Kohledampfabscheidung mit thermischen Gradienten erhält.

7. Vorrichtung nach einem dere vorhergehenden Ansprüche, dadurch gekennzeichnet, daß eine weitere Komponente wie oben erwähnt, als anderer, damit verbundender Bestandteil wirkt.

## Revendications

1. Dispositif d'endoprothèse d'articulation d'os, comprenant un élément dont une partie (12, 21) est detinée à une fixation à l'os, dont une autre partie (11, 22) est destinée à un contact mutuel d'articulation avec une autre partie intégrante de l'articulation, et qui est essentiellement entièrement poreuse, dispositif caractérisé en ce que la porosité dans ladite partie (12, 21) est supérieure à celle existant dans l'autre partie (11, 22), respectivement, pour favoriser la formation, par anastomose, d'une matière osseuse et fibrocartilagineuse.

2. Dispositif selon la revendication 1, caractérisé en ce que ladite porosité implique une variation du diamètre des pores, dans lesdites un et autre parties, à l'intérieur d'intervalles respectifs allant de 10 à 250 μm et allant de sensiblement 0 à 150 μm.

3. Dispositif selon la revendication 1 ou 2, caractérisé en ce que ledit élément est constitué d'une matière à armature de fibres, cette armature étant agencée de façon à se conformer à un modèle prédéterminé, lequel simule celui de la structure trabéculée de l'os remplacé par ledit élément.

4. Dispositif selon la revendication 1 2 ou 3, caractérisé en ce que ledit élément est constitué de carbone à armature de fibres de carbone.

5. Dispositif selon la revendication 4, dans lequel ledit élément est constitué de tronçons initialement séparés, respectivement, pour former lesdites une et autre parties, ces tronçons étant soumis à des degrés respectivement différents de dépôt isotherme de vapeur de carbone pour permettre l'obtention d'une porosité différente de façon correspondante, et lesdits tronçons, étant ensuite reliés ensemble.

6. Dispositif selon la revendication 4, caractérisé en ce que ledit élément est formé par dépôt de vapeur de carbone avec gradient thermique de manière à présenter ladite porosité variable.

7. Dispositif selon l'une quelconque des revendications précédentes, caractérisé en ce qu'il comporte un autre élément, comme indiqué ci-dessus destiné à jouer le rôle de ladite autre partie intégrante.

Fig. 1

Fig. 2

Fig. 3

Fig. 4